# EUROPEAN PATENT APPLICATION

(11) **EP 4 201 962 A1**
(43) Date of publication of application: **28.06.2023**
(21) Application number: 21858634.5
(22) Date of filing: 20.08.2021
(51) Int. Cl.: C07K 16/46

(54) **ANTIBODY FRAGMENT CONSISTING OF HEAVY CHAIN AND LIGHT CHAIN CONSTANT REGIONS IN WHICH GAMMA CONSTANT REGION (C?1) AND EPSILON CONSTANT REGION (C?2-4) ARE FUSED, AND USE THEREOF**

(30) Priority: 21.08.2020 KR 20200105462; 19.08.2021 KR 20210109364
(71) Applicant: Ajou University Industry-Academic Cooperation Foundation, Gyeonggi-do 16499 (KR)
(72) Inventor: KWON, Myung-Hee, Yongin-si Gyeonggi-do 16936 (KR); KIM, Minjae, Yongin-si Gyeonggi-do 16910 (KR); LEE, Jeonghyun, Suwon-si Gyeonggi-do 16711 (KR); CHOI, Juho, Hwaseong-si Gyeonggi-do 18321 (KR); SEO, Youngsil, Suwon-si Gyeonggi-do 16501 (KR)
(74) Representative: Mewburn Ellis LLP
(86) International application number: PCT/KR2021/011105
(87) International publication number: WO 2022/039547

(57) **Abstract**

The present invention relates to an antibody fragment consisting only of the constant regions of an antibody and, more specifically, to an antibody fragment having no variable region of an antibody and consisting only of heavy chain (C_{H}) and light-chain (C_{L}) constant regions. For example, the present invention relates to: an antibody fragment (IgCw-γ1ε₂₋₄/κ) consisting of heavy chain and light-chain constant regions in which a gamma constant region ((Cγ1) and an epsilon constant region (Cε₂₋₄) are fused; a nucleic acid encoding same; a kit comprising same; and use thereof. To evaluate the efficacy of antibodies in the development of new antibody drugs and the like, a control, i.e., a reference antibody is required, and existing reference antibodies cannot be completely free from non-specific binding, i.e., off-target effects due to reaction with other antigens, and also have low purification yield. Meanwhile, when the recombinant protein IgCw-γ1ε₂₋₄/κ consisting only of the constant regions of an antibody, according to the present invention, is used, the problems of existing reference antibodies can be overcome, and the recombinant protein can also be used as an Fc epsilon receptor inhibitor for inhibiting allergic responses. Therefore, IgCw-γ1ε₂₋₄/κ according to the present invention can be used in various ways in the biomedical science field.

## Description

### [Technical Field]

The present invention relates to an antibody fragment including only antibody constant regions and more particularly, to an antibody fragment including only heavy-chain (CH) and light-chain (CL) constant regions without antibody variable regions, for example, an antibody fragment including constant regions (CL) of heavy- and light-chains in which gamma constant regions (Cγ1) are fused with epsilon constant regions (Cε2-4) (called "IgCw-γ1ε2-4/κ", abbreviated IgCw-γεκ), a nucleic acid encoding the same, a kit including the same, and the use thereof.

### [Background Art]

In order to prove *in vitro* and *in vivo* efficacies of recombinant antibodies, reference antibodies, that is, control antibodies that do not bind to the target molecule (antigen) of antibodies used to evaluate the efficacies are required. The reference antibodies can be produced from cultures of cells isolated from polyclonal antibodies or transfected with expression constructs encoding the heavy and light-chain regions of antibodies.

Although it is expected that the reference antibody does not bind to the target molecule to which the antibody used to evaluate efficacy is bound, the possibility of cross-reactivity in which a noise positive signal is generated, particularly, the possibility of off-target effects due to reaction with other antigens, cannot be completely ruled out because the reference antibody has a variable region involved in binding to an antigen.

Meanwhile, immunoglobulin E (IgE) is an antibody present in very low concentrations in the blood. Measurement of the concentration of IgE in the blood is essential for diagnosis of IgE-related immune diseases and tracking and management of therapeutic effects. A reference IgE protein is absolutely necessary for quantification of IgE. In addition, IgE protein is indispensable for various immunological qualitative experiments.

Currently commercially available human IgE proteins include as follows: 1) monoclonal IgE purified from the blood of patients with high levels of IgE in the blood (e.g., IgE myeloma patients) (e.g., products from Abcam, Athens Research & Technology, MyBioSource, Fitzgerald Industries International, Molecular Innovations, and Merck Millipore), 2) monoclonal IgE purified from the conditioned culture of hybridoma cells formed by fusing B cells derived from healthy individuals with myeloma cells, and 3) monoclonal IgE purified from the culture medium of cell lines obtained by transfection of IgE genes.

These IgE antibodies are at least 100 times more expensive than IgG and have no non-specific binding affinity to unknown antigens in IgE quantification experiments and various immunological experiments because they include variable regions (VH and VL). There is an international standard IgE [WHO IgE International standard, the most recent is the 3rd International Reference Preparation (IRP), coded 11/234] for public purposes used to quantify the total IgE in the blood. The WHO IRP 11/234 is a lyophilizate as an ampoule containing blood plasma (or serum) collected from each of patients with high concentrations of IgE in the blood in various countries, is considered a biohazard due to blood components contained therein and thus should be carefully used in the laboratory, is polyclonal IgE and is problematic because the possibility of non-specific binding to an unknown antigen cannot be ruled out.

Accordingly, the present inventors attempted to develop a novel antibody fragment that can replace the IgE reference antibody while avoiding the disadvantages of the previously known IgE reference antibody. In addition, the present inventors attempted to develop a molecule that can be used instead of IgE in quantitative experiments to measure the concentration of IgE as well as in qualitative experiments to inhibit the FcεR-IgE interaction since it has a high production yield and can bind to the Fc epsilon receptor (FcεR).

As a result, the present inventors developed a molecule including only constant regions without variable regions, especially an antibody fragment (IgCw-γε_{K}) including a heavy-chain (Cγ1-hinge-Cε₂₋₄) in which a gamma constant region (Cγ1)-hinge is fused with an epsilon constant region (Cε2-4), and a kappa constant region (C_{κ}) of the light-chain. The present inventors found that this IgCw-γεκ molecule can be used as an IgE substitute in IgE concentration measurement experiments and immunology research based on FcεR-IgE interaction and can suppress IgE-mediated hypersensitivity diseases, thus completing the present invention based thereon.

The information disclosed in this Background section is provided only for enhancement of understanding of the background of the present invention, and therefore it may not include information that forms the prior art that is already obvious to those skilled in the art.

### [Disclosure]

Therefore, it is one object of the present invention to provide an antibody fragment including only heavy-chain (C_{H}) and light-chain (C_{L}) constant regions without antibody variable regions.

It is another object of the present invention to provide a nucleic acid encoding the antibody fragment.

It is another object of the present invention to provide a kit including the antibody fragment.

It is another object of the present invention to provide a composition for evaluating antibody efficacy containing the antibody fragment.

It is another object of the present invention to provide a composition for measuring an antibody concentration containing the antibody fragment.

It is another object of the present invention to provide a composition for suppressing allergic reactions containing the antibody fragment.

It is another object of the present invention to provide a composition for inhibiting an IgE-mediated-autoimmune reaction containing the antibody fragment.

In accordance with one aspect of the present invention, the above and other objects can be accomplished by the provision of an antibody fragment including a heavy-chain constant region fragment of an IgG antibody and a heavy-chain constant region fragment of an IgE antibody, and a light-chain constant region fragment linked to the heavy-chain constant region fragment.

In accordance with another aspect of the present invention, provided is a nucleic acid encoding the antibody fragment.

In accordance with another aspect of the present invention, provided is a kit including the antibody fragment.

In accordance with another aspect of the present invention, provided is a composition for evaluating antibody efficacy containing the antibody fragment.

In accordance with another aspect of the present invention, provided is a composition for measuring an antibody concentration containing the antibody fragment.

In accordance with another aspect of the present invention, provided is a composition for suppressing allergic reactions containing the antibody fragment.

In accordance with another aspect of the present invention, provided is a composition for inhibiting an IgE-mediated-autoimmune reaction containing the antibody fragment.

### [Description of Drawings]

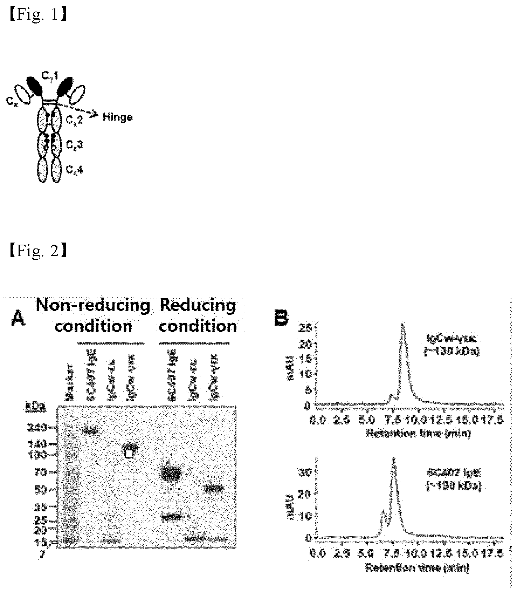

FIG. 1 illustrates the structure of an IgCw-γ1ε₂₋₄/κ (simply referred to as "IgCw-γεκ)" protein and shows an antibody fragment (about 130 kDa) including a Cγ1-hinge region and Cε2-Cε3-Cε4 of a human antibody.
FIG. 2 illustrates the results of analysis of the purity and integrity of the purified antibody proteins, and shows the results of analysis of purity and integrity of the antibody proteins purified from the conditioned culture of HEK293f cells transfected with each antibody gene-expressing vector, identifying that IgCw-γεκ has a molecular weight of about 130 kDa, as expected, wherein A shows the result of Coomassie staining after SDS-PAGE and B shows the result of size exclusion chromatography.

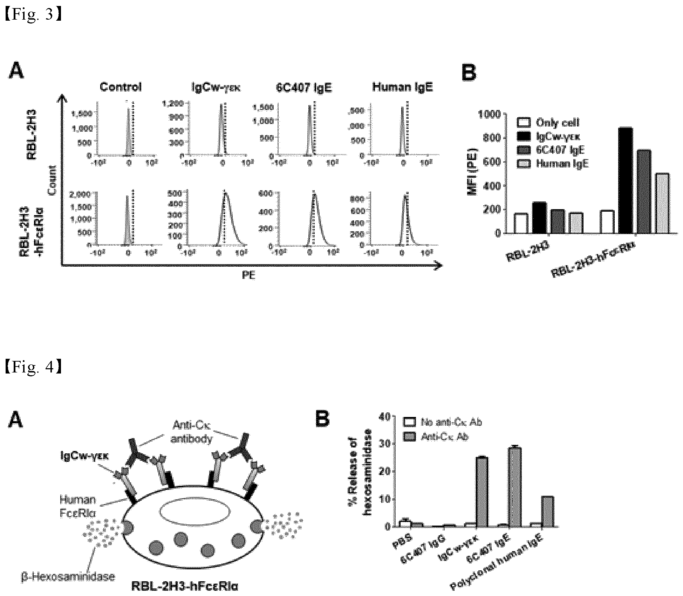

FIG. 3 shows the result of analysis of the binding between IgCw-γεκ and FcεR, and more particularly, the result of flow cytometry elucidating that the IgCw-γεκ antibody fragment protein can bind to the high-affinity human Fcε receptor (FcεR1) expressed on the surface of RBL-2H3-FcεRIα cells, comparable to control IgE.
FIG. 4 shows the result of identification of the degranulation reaction by cross-linking of IgCw-γεκ and elucidates that when RBL-2H3-FcεRIα cells were sensitized by treatment with IgCw-γεκ and then treated with anti-Cκ antibody (to induce the cross-linking of FcεR1), IgCw-γεκ can induce beta-hexosaminidase release (degranulation reaction) to a level similar to that of full-size IgE as a control, wherein A shows the experimental design, B shows the experimental result.

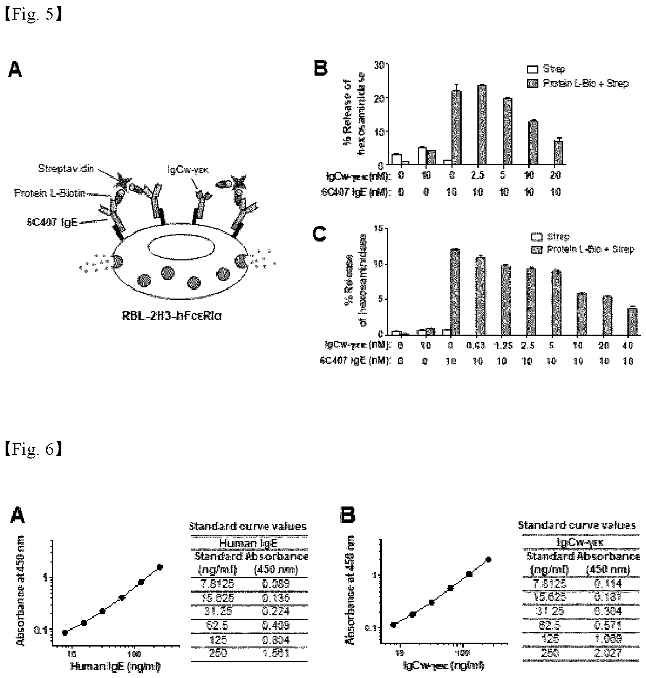

FIG. 5 shows the results of inhibition of the degranulation reaction in IgE-sensitized cells by IgCw-γεκ and elucidates that IgCw-γεκ can inhibit beta-hexosaminidase release (degranulation reaction) in RBL-2H3-FcεRIα cells by 6C407 IgE+Protien-L-Biotin+Streptavidin, wherein A shows the experimental design, B shows the result of measurement of the degree of beta-hexosaminidase release in RBL-2H3-FcεRIα cells treated with a mixture of 6C407 IgE and IgCw-γεκ, and C shows the result of the degree of beta-hexosaminidase release from RBL-2H3-FcεRI cells pre-treated with 6C407 IgE and then treatment with IgCw-γεκ.
FIG. 6 shows IgCw-γεκ as a reference molecule to quantify human IgE antibodies and more particularly, shows the result of ELISA elucidating that IgCw-γεκ protein can be used as a reference to quantify an IgE antibody, wherein A represents a standard curve created using 6C407 IgE and B represents a standard curve created using IgCw-γεκ.

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

Production of an IgCw-εκ antibody fragment including only constant regions of a human IgE antibody was attempted. However, IgCw-εκ including only the constant regions of the epsilon heavy-chain and the kappa light-chain did not form a disulfide bond between the heavy-chain and light-chain constant regions, and allows for purification of only the kappa light-chain constant region, without binding to the heavy-chain constant region, when purified with the binding Kappa XP-agarose bound to the human kappa light-chain constant region.

Finally, the antibody fragment IgCw-γεκ including the constant regions of the heavy- and light-chains in which the gamma constant region (Cγ1) -hinge is fused with the epsilon constant region (Cε₂₋₄) has a disulfide bond normally formed between the constant regions and the expression level and has expression level and purification yield similar to that of the control 6C407 IgE. As such, fusion of the gamma constant region (Cγ1) with the epsilon constant region (Cε₂₋₄) enables structural stability and high purification yield of the IgCw-γεκ protein.

In addition, the present inventor developed a novel antibody fragment (referred to as "IgCw-γ1ε₂₋₄/κ", simply referred to as "IgCw-γεκ") that can be used as an alternative to the previously known IgE reference antibodies while avoiding the disadvantages of the IgE reference antibodies. IgCw-γεκ is an antibody fragment molecule including two Cγ1-hinge-Cε₂₋₄ hybrid heavy-chains and two Cκ light-chains, which includes only constant regions without variable regions.

Based thereon, in one aspect, the present invention is directed to an antibody fragment including a heavy-chain constant region fragment of an IgG antibody and a heavy-chain constant region fragment of an IgE antibody, and a light-chain constant region fragment linked to the heavy-chain constant region fragment.

The antibody fragment according to the present invention is obtained by developing, as a novel antibody format, an antibody fragment which includes an IgG antibody heavy-chain constant region fragment and an IgE antibody heavy-chain constant region fragment without an antibody variable region, and a light-chain constant region fragment linked to the heavy-chain constant region fragment, and identifying that IgCw-γεκ having such a novel structure has various advantages and can be widely used in clinical fields such as antibody efficacy testing.

Unfragmented antibodies include two heavy-chains and two light-chains linked by disulfide bonds. Each single light-chain is linked to one of the heavy-chains by a disulfide bond. An antibody heavy-chain portion has, at the N-terminus, a variable domain (VH) followed by a plurality of constant domains (3 or 4 constant domains, C_{H}1, C_{H}2, C_{H}3 and C_{H}4, depending on the type of antibody). Each light-chain portion has a variable region (VL) at the N-terminus thereof and a constant region (CL) at the other terminus (C-terminus) thereof, the light-chain constant region is aligned with the first constant region (CH1) of the heavy-chain, and the light-chain variable region (V_{L}) is aligned with the heavy-chain variable region (V_{H}).

The constant regions refer to the entirety of antibody domains other than the variable regions. The constant regions are not directly involved in the binding of an antibody to the target antigen thereof, but are involved in various effector functions *in vivo.* The heavy- and light-chain constant regions encompass those derived from IgA, IgD, IgE, IgG and IgM as well as those derived from IgY, IgW and IgNAR.

In one embodiment, the IgG may include IgG1, IgG2, IgG3, and IgG4 as subtypes.

In one embodiment, the heavy-chain constant region fragment of the IgG antibody may include Cγ1. The heavy-chain constant region fragment of the IgE antibody may include at least one selected from the group consisting of Cε2, Cε3 and Cε4.

In a specific embodiment, the antibody fragment according to the present invention includes an IgG antibody heavy-chain constant region fragment Cγ1, and IgE antibody heavy-chain constant region fragments Cε2, Cε3 and Cε4, and the IgG antibody heavy-chain constant region fragment may be linked to the IgE antibody heavy-chain constant region fragment from the N-terminus to the C-terminus in the form of Cγ1-Cε2-Cε3-Cε4.

The IgG antibody heavy-chain constant region fragment may be linked to the IgE antibody heavy-chain constant region fragment through a hinge.

In one embodiment, the light-chain constant region may include Cκ or Cλ. The light-chain constant region fragment linked to the heavy-chain constant region fragment may be bonded thereto, for example, by a disulfide bond or via a peptide linker.

Recombinant antibody formats often face difficulties due to low purification yields that can affect usefulness and cost. The production yield of an antibody depends on the characteristics (amino acid sequences) of the heavy-chain variable region (V_{H}) and the light-chain variable region (V_{L}). Thus, the production yield of a recombinant antibody may be quite variable depending on the amino acid sequence of the variable domain (V domain) even in well-established manufacturing processes. In one embodiment of the present invention, it was found that the antibody fragment including only the antibody constant region according to the present invention can be produced in high yield.

In another aspect, the present invention is directed to a nucleic acid encoding the antibody fragment. The antibody fragment can be produced recombinantly. The nucleic acid is isolated and inserted into a replicable vector, followed by further cloning (amplification of DNA) or further expression. Based on this, in another aspect, the present invention is directed to a vector including the nucleic acid.

The term "nucleic acid" is intended to encompass both DNA (gDNA and cDNA) and RNA molecules, and a nucleotide, which is a basic constituent unit of a nucleic acid, includes naturally derived nucleotides as well as analogues, wherein sugar or base moieties are modified. The sequence of the nucleic acid encoding heavy- and light-chain variable regions of the present invention can vary. Such variation includes addition, deletion, or non-conservative or conservative substitution of nucleotides.

The DNA encoding the antibody fragment can be easily separated or synthesized using conventional procedures (for example, using an oligonucleotide probe capable of specifically binding to DNA encoding heavy- and light-chains of the antibody) . A variety of vectors are obtainable. Vector components generally include, but are not limited to, one or more of the following components: signal sequences, replication origins, one or more marker genes, enhancer elements, promoters and transcription termination sequences.

As used herein, the term "vector" refers to a means for expressing target genes in host cells and includes plasmid vectors, cosmid vectors, and viral vectors such as bacteriophage vectors, adenovirus vectors, retroviral vectors and adeno-associated viral vectors. The polynucleotide encoding the antibody in the vector is operably linked to a promoter.

The term "operably linked" means a functional linkage between a nucleic acid expression regulation sequence (e.g., promoter, signal sequence or array of transcription regulator binding sites) and another nucleic acid sequence, and enables the regulation sequence to regulate the transcription and/or translation of the other nucleic acid sequence.

When a prokaryotic cell is used as a host, it generally includes a potent promoter capable of conducting transcription (such as a tac promoter, lac promoter, lacUV5 promoter, lpp promoter, pLλ, promoter, pRλ promoter, rac5 promoter, amp promoter, recA promoter, SP6 promoter, trp promoter, or T7 promoter), a ribosome-binding site for initiation of translation, and a transcription/translation termination sequence. In addition, for example, when a eukaryotic cell is used as a host, it includes a promoter (e.g., a metallothionein promoter, a β-actin promoter, a human hemoglobin promoter and a human muscle creatine promoter) derived from the genome of mammalian cells, or a promoter derived from a mammalian virus such as an adenovirus late promoter, vaccinia virus 7.5k promoter, SV40 promoter, cytomegalovirus (CMV) promoter, HSV tk promoter, mouse mammary tumor virus (MMTV) promoter, HIV LTR promoter, Moloney virus promoter, Epstein-Barr virus (EBV) promoter, and Rous sarcoma virus (RSV) promoter, and generally has a polyadenylation sequence as a transcription termination sequence.

Optionally, the vector may be fused with another sequence in order to facilitate purification of the antibody expressed therefrom. The sequence to be fused includes, for example, glutathione S-transferase (Pharmacia, USA), maltose-binding protein (NEB, USA), FLAG (IBI, USA), 6x His (hexahistidine; Quiagen, USA) and the like.

The vector includes antibiotic resistance genes commonly used in the art as selectable markers, and examples thereof include genes conferring resistance to ampicillin, gentamycin, carbenicillin, chloramphenicol, streptomycin, kanamycin, geneticin, neomycin and tetracycline.

In another aspect, the present invention is directed to a cell transformed with the above-mentioned vector. The cell used to produce the antibody of the present invention may be a prokaryote, yeast or higher eukaryotic cell, but is not limited thereto.

Prokaryotic host cells such as *Escherichia coli*, the genus *Bacillus,* such as *Bacillus subtilis* and *Bacillus thuringiensis, Streptomyces* spp., *Pseudomonas* spp. (for example, *Pseudomonas putida)*, *Proteus mirabilis* and *Staphylococcus* spp. (for example, *Staphylococcus carnosus)* can be used.

Interest in animal cells is the greatest, and examples of useful host cell lines include, but are not limited to, COS-7, BHK, CHO, CHOK1, DXB-11, DG-44, CHO/-DHFR, CV1, COS-7, HEK293, BHK, TM4, VERO, HELA, MDCK, BRL 3A, W138, Hep G2, SK-Hep, MMT, TRI, MRC 5, FS4, 3T3, RIN, A549, PC12, K562, PER.C6, SP2/0, NS-0, U20S, and HT1080.

In another aspect, the present invention is directed to a method of producing the antibody fragment including: (a) culturing the cell; and (b) recovering an antibody fragment from the cultured cell.

The cell can be cultured in various media. Any commercially available medium can be used as a culture medium without limitation. All other essential supplements well-known to those skilled in the art may be included in appropriate concentrations. Culture conditions such as temperature and pH are conventionally used with host cells selected for expression, as will be apparent to those skilled in the art.

The recovery of the antibody fragment can be carried out, for example, by centrifugation or ultrafiltration to remove impurities and purification of the resulting product using, for example, affinity chromatography. Other additional purification techniques such as anion or cation exchange chromatography, hydrophobic interaction chromatography and hydroxyapatite (HA) chromatography may be used.

In another aspect, the present invention is directed to a kit including the antibody fragment. The kit may include a container containing the antibody fragment and a container containing another reagent or sample.

The kit suitably includes at least one container such as a bottle or tube, and each container includes independent components used in the method of the present invention. Those skilled in the art can easily dispense the required formulation in the container.

In another aspect, the present invention is directed to a composition for evaluating antibody efficacy containing the antibody fragment.

In experiments demonstrating the effect of an antigen-specific antibody, a reference (or irrelevant isotype control) antibody is usually used as a negative control to distinguish non-specific background signals from antigen-specific antibody signals. However, conventional reference antibodies may still form unwanted noise signals due to cross-reactivity that was not known clearly.

The antibody fragment according to the present invention may be a better reference antibody in many experimental environments and situations compared to irrelevant IgE controls since it has no antigen-binding ability and can exclude the possibility of unexpected cross-reactivity.

In another aspect, the present invention is directed to a composition for measuring an antibody concentration containing the antibody fragment. The antibody fragment according to the present invention may be used as a reference for measuring immunoglobulin concentration in a biological sample.

In another aspect, the present invention is directed to a composition for suppressing allergic reactions containing the antibody fragment.

Most allergic diseases are caused by an excessive immune response of immunoglobulin E (IgE). IgE is an antibody present at a very low concentration in serum under normal conditions. IgE is generally produced by harmless antigens and often increases even without specific stimulation. In this case, allergic diseases may occur. Abnormally increased IgE may bind to the high-affinity IgE Fc receptor (FcεRI) expressed on the surface of mast cells and basophils. When antigens (mainly innocuous antigens) bind to several IgE molecules at the same time in the state where IgE molecules are bound to FcεRI, cross-linking between FcεRI receptors occurs, and signal transduction into mast cells and basophil granulocytes occurs, resulting in activation. As a result of the cell activation, the mast cells or basophil granulocytes release chemical mediators such as histamines, leukotrienes, prostaglandins, bradykinins, and platelet-activating factors. The release of these chemical mediators causes allergic symptoms. The antibody fragment according to the present invention can be used as a blocker of the Fc epsilon receptor (FcεRI) for suppressing allergic reactions.

In another aspect, the present invention is directed to a composition for inhibiting an IgE-mediated-autoimmune reaction containing the antibody fragment.

One of the pathogenesis mechanisms of autoimmune diseases such as systemic lupus erythematosus (SLE) is the secretion of large amounts of inflammatory cytokines (IFN-α, TNF, IL-6) by plasmacytoid dendritic cells (pDCs). Like mast cells, pDC cells express FcεRI on the surface thereof and can secrete 1,000 times more IFN-α than other cells upon activation. When a complex (immunocomplex) of an IgE isotype autoantibody and a DNA antigen binds to FcεRI on the surface of pDC, the immune complex enters the cells through FcεRI. The immune complex entering the cells stimulates intracellular receptors such as toll-like receptor (TLR)-7 and -9 to activate pDC cells, resulting in the secretion of large amounts of inflammatory cytokines and exacerbation of disease. The antibody fragment according to the present invention can be used as a blocker of the Fc epsilon receptor (FcεRI) to inhibit autoimmune responses mediated by IgE autoantibodies.

Hereinafter, the present invention will be described in more detail with reference to the following examples. However, it will be obvious to those skilled in the art that the following examples are provided only for illustration of the present invention and should not be construed as limiting the scope of the present invention based on the subject matter of the present invention.

### Example 1: Preparation of sample

### Example 1-1: Plasmid vector

DNA fragments in which human heavy-chain constant regions Cγ1 and ε2-4 are hybridized and encoded were cloned between the restriction sites *NcoI* and *BamHI* of the KV10-IgCWγκ vector in order to form a KV10-IgCw-γεκ vector that simultaneously expresses human Cγ1/ε₂₋₄ and human Cκ, each having a leader sequence by which two CMV promoters each regulate genes.

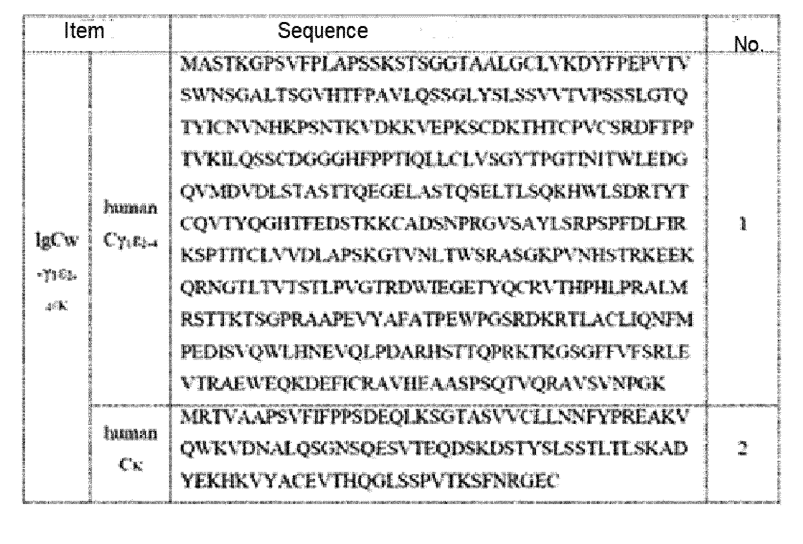

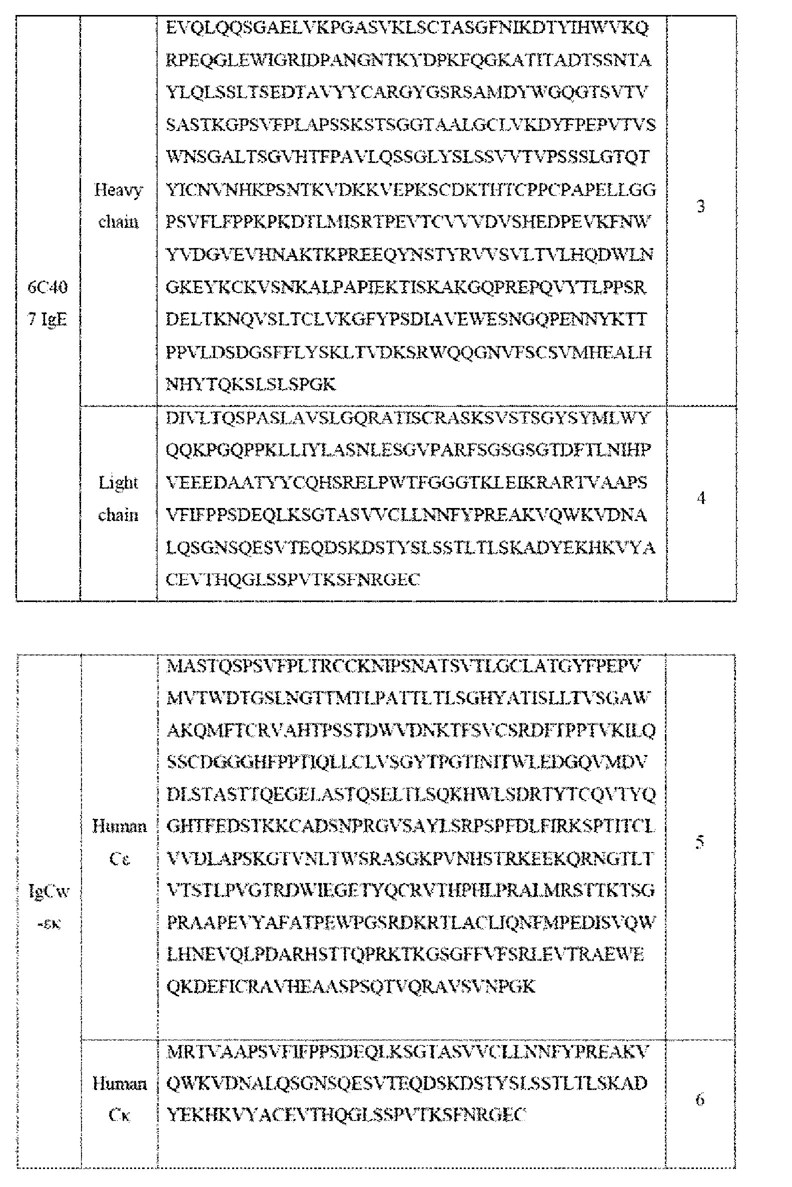

DNA fragments encoding the V_{H} and V_{K} domains were cloned between the *MluI* and *NhEI* restriction sites of the KV12-HL vector and between the *DraIII* and *BstWI* restriction sites, respectively, in order to form plasmid vectors for chimeric IgE (6C407, 3D8) expression.

NA fragments encoding the human heavy-chain constant region (Cε1-4) were cloned between the restriction sites *NcoI* and *BamHI* of the KV10-IgCWγκ vector in order to form KV10-IgCw-εκ vectors that simultaneously express human Cε1-4 and human Cκ, each having a leader sequence, by which two CMV promoters each regulate genes.

A DNA fragment encoding human FcεRIα was cloned between restriction sites *EcoI* and *BamHI* of the pCDH-CMV-MCS-EF1-Puro vector in order to form a lentiviral vector expressing human FcεRIα.

### Example 1-2: Preparation of immunoglobulin protein using HEK293F cells

The Freestyle^{™} 293-F cell line (Thermo Fisher Scientific) adapted to grow by suspension culture under serum-free conditions was used to produce an antibody.

To adjust the number of cells to 2×10⁶ cells/ml during transfection, 100 ml of FreeStyle HEK293F cells were cultured at 1×10⁶ cells/ml in a 500 ml flask (Corning Cat# 431145) 24 hours before transfection. The FreeStyle 293-F cells were shake-cultured in serum-free Freestyle 293 medium (Invitrogen Cat# 12338) in an 8% CO₂, 37°C incubator at 130 rpm. To express 6C407 IgE, IgCw-εκ, and IgCw-γεκ proteins, 200 µg of each gene-encoded KV plasmid and 400 µg of polyethylenimine (PEI) reagent (Polyscience Cat# 23966-2) were allowed to stand in 5 ml of Freestyle 293 medium at room temperature for 10 minutes. Then, 5 ml of the medium containing the DNA was filtered through a 0.22 um syringe filter (Millipore Cat# SLGV033RB), mixed with 5 ml of a medium containing polyethyleneimine, and allowed to stand at room temperature for 10 minutes. Finally, 100 ml of Freestyle 293-F cells were transiently transfected to adjust the concentration of polyethyleneimine to 4 ug/ml. 7 days later, the culture medium was centrifuged at 4°C at 400 g for 20 minutes to obtain the supernatant, filtered through a 0.45 um cellulose acetate filter (Sartorius Cat# 11106-47-N) and then was allowed to pass through a CaptureSelect^{™} KappaXP (Thermo Fisher Scientific; Cat# 2943212005) column or a CaptureSelect^{™} IgG-CH1 (Thermo Fisher Scientific; Cat# 194320005) column. After the column was washed with PBS (phosphate-buffered saline, pH 7.4), proteins were eluted with 0.1M glycine solution (glycine-HCl, pH 3.0). The eluted protein was concentrated using Vivaspin 20 (molecular cutoff 50,000, Sartorius Cat# VS2032) (4°C, 1500 g).

The concentration of the purified protein was determined using the absorbance and extinction coefficient at 280 nm. The molar extinction coefficients at 280 nm were 1.49 for 6C407 IgE, 1.34 for IgCw-εκ, and 1.17 for IgCw-γεκ, respectively, and were calculated from the respective amino acid sequences through the web page http://web.expasy.org/protparam/.

### Example 1-3: Cell culture

RBL-2H3 (ATCC^{®} number: CRL-2256^{™}) cell line derived from rat basophil leukemia cells and human embryonic kidney 293 cell line HEK293T (ATCC^{®} number: CRL-3216^{™}) were cultured in DMEM (Dulbecco's Modified Eagle's Medium, Welgene Inc.). The DMEM used herein was supplemented with 10% fetal bovine serum (Sigma Inc.), 100 U/ml penicillin (Welgene Inc.), and 100 ug/ml streptomycin (Streptomycin, Welgene Inc.). All cells were cultured in a 5% CO₂, 37°C incubator.

### Example 1-4: Cell construction

The human embryonic kidney 293 cell line HEK293T was used to produce a supernatant containing lentivirus expressing FcεRIα.

To adjust the number of cells to 3×10⁶ cells/ml during transfection, HEK293T cells were cultured at a density of 1.5×10⁶ cells/well in 4 ml of DMEM in a 60 mm² culture dish (SPL; Cat# 11060) 24 hours before transfection. After 24 hours, the medium was discarded, and 3.8 ml of DMEM was mixed with 200 ul of Opti-MEM^{™} (Thermo Fisher Scientific; Cat# 31985070) supplemented with 4 µg of pCDH plasmid encoding the gene, 3 µg of gag/pol (Addgene cat# 14887), 1 µg of VSV-G (Addgene cat# 14888) and 16 µg of polyethylenimine (PEI) reagent (Polyscience Cat# 23966-2) to express FcεRIα. The result was cultured at 5% CO₂ and 37°C for 16 hours, the medium was discarded, and culture was performed in 4 ml of fresh DMEM 24 hours. After 24 hours, the medium was collected and centrifuged at 2,000 rpm for 3 minutes to obtain a supernatant, which was filtered through a 0.45 um syringe filter (Syringe filter, Millipore Cat# SLHV033RS) to obtain a supernatant containing lentivirus.

The supernatant containing lentivirus to express a receptor recognizing human IgE in RBL-2H3 cells was prepared.

To adjust the number of cells to 2×10⁶ during infection, 24 hours before infection, RBL-2H3 cells were cultured at 1×10⁶ cells/well in a 60 mm² culture dish in 4 ml of DMEM. After 24 hours, the medium was removed, the cells were removed once with PBS (phosphate-buffered saline, pH 7.4), mixed with 3 ml of DMEM and 1 ml of the supernatant containing lentivirus, and 10 ug/ml polybrene (Sigma-Aldrich; Cat# H9268) was added thereto. After culturing for 24 hours, the medium was removed and 4 ml of DMEM was added thereto, followed by culturing for another 12 hours. Then, the cells were cultured in 5 ug/ml puromycin (Sigma-Aldrich; Cat# 540411) .

### Example 2: Analysis method

### Example 2-1: Flow cytometry

In order to determine whether or not receptors are expressed in RBL-2H3-hFcεRIα cells expressing human Fcε receptors, cells (1×10⁶ cells) were washed with cold PBS and then fixed in 4% paraformaldehyde diluted in PBS at room temperature for 20 minutes. The detection antibody APC-conjugated mouse anti-human Fc epsilon RI Ab (Abcam; Cat# 155369) was diluted in buffer S (0.5% BSA diluted in PBS, 2 mM EDTA, pH 8.5). The result was allowed to stand at 4°C for 1 hour and then was washed three times with cold PBS. The result was analyzed through a FACS CantoII analyzer (BD Biosciences).

In order to determine whether or not IgCw-γεκ binds to the Fcε receptor expressed on the cell surface, FcεR- RBL-2H3 and FcεR+ RBL-2H3-hFcεRIα cells (1×106 cells) were treated with immunoglobulin proteins at a final concentration of 1 µM at 37°C for 3 hours. The cells were washed with cold PBS, and fixed with 4% paraformaldehyde diluted in PBS for 20 minutes at room temperature. For RBL-2H3 and RBL-2H3-hFcεRIα cells, goat anti-Human IgE (ε-chain specific) antibody (Sigma-Aldrich; Cat# I6284) as a primary antibody and PE-conjugated donkey anti-goat IgG antibody (Abcam; Cat# Ab7004) as a secondary antibody were diluted in buffer S. The cells were washed three times with cold PBS whenever they were allowed to stand (4°C, 1 hour) . Each sample was analyzed through a FACS CantoII analyzer (BD Biosciences).

### Example 2-2: Enzyme-linked immunosorbent assay (ELISA) to measure IgE concentration

An enzyme-linked immunosorbent assay was performed to form a standard curve of full-size IgE and IgCw-γεκ included in the kit using human IgE ELISA Ready-SET-Go (Invitrogen; Cat# 88-50610) kit to quantify human IgE. The experimental method followed the instructions of the kit.

The wells of a 96-well polystyrene plate were coated with a coating antibody at 4°C for 16 hours. Then, at each standing stage (room temperature), the cells were washed four times with TBS (TRIS-buffered saline; 50 mM TRIS-Cl, 50 mM NaCl, pH 7.4) containing 0.05% Tween-20. In order to block the binding of non-specific antibodies, the cells were treated with 3% BSA (bovine serum albumin) at room temperature for 2 hours. Human polyclonal IgE and IgCw-γεκ were serially diluted 2-fold from a starting concentration of 250 ng/ml, and the wells were treated with the dilution at room temperature for 2 hours and treated with a detection antibody at room temperature for 1 hour. Finally, the reaction substrate solution was added to each well, and the absorbance was measured at 450 nm using a microplate reader (Molecular Devices Inc.).

To measure the concentration of an antibody of an unknown concentration, the wells of a 96-well polystyrene plate were coated with the coated antibody at 4°C for 16 hours. The wells were treated with monoclonal 6C407 IgE or 3D8 IgE, and then with the detection antibody (both treated at room temperature for 1 hour). Concentrations of IgE samples were determined by interpolation of Y-axis values in two different standard curves created using human IgE and IgCw-γεκ of known concentrations, respectively.

### Example 2-3: Beta hexosaminidase release assay (degranulation assay)

Beta-hexosaminidase secretion was measured to determine whether or not the complex in which the IgE binds to the Fcε receptor exhibited degranulation. To adjust the number of RBL-2H3-hFcεRIα cells to 5×10⁵ cells/well, 2.5×10 5 cells/well were cultured in 400 µl of DMEM in a 24-well plate (SPL; Cat# 30024) before 24 hours. After 24 hours, in order to sensitize IgE, 10 nM IgE was cultured in 400 µl of DMEM containing no fetal bovine serum, penicillin and streptomycin for 3 hours. The IgE was removed and then the cells were washed twice with 500 ul of Siraganian buffer (119 mM NaCl, 5 mM KCl, 5.6 mM glucose, 0.4 mM MgCl₂, 25 mM PIPES, 40 mM NaOH, 1 mM CaCl₂, 0.1% BSA, pH 7.2). The result was allowed to stand in 160 µl Siraganian buffer in a 5% CO₂, 37°C incubator for 10 minutes. Goat anti-Human Kappa Light chain antibody (Invitrogen; Cat# 31129) was mixed with Siraganian buffer to obtain a final concentration of 10 µg/ml, and then 20 ul of the resulting mixture was added to each well, followed by incubation for 20 minutes. 0.1% Triton X-100 used as a control group was allowed to stand for 1 hour in a 5% CO₂, 37°C incubator. After incubation in a 5% CO₂, 37°C incubator for 20 minutes, 50 µl of the result was added to each well of a 96-well polystyrene plate, and 50 µl of 1 mM p-NAG (p-nitrophenyl N-acetyl-beta-D-glucosamine in 0.1 M citrate buffer, pH 4.5) was added thereto. After treatment at 37°C for 1 hour, 200 µl of stop solution (0.1 M Na₂CO₃ /NaHCO₃, pH 10.0) was added to each well and absorbance at 405 nm was measured using a microplate reader (Molecular Devices Inc.).

### Example 2-4: Measurement of beta-hexosaminidase secretion to determine blocker role of Fcε receptor

Beta-hexosaminidase secretion was measured to determine whether or not the complex in which IgE binds to Fcε receptors acts as a blocker of Fcε receptors, which serves to prevent degranulation caused by other IgE. To adjust the number of RBL-2H3-hFcεRIα cells to 5×105 cells/well, 2.5×10⁵ cells/well were cultured in 400 µl of DMEM in a 24-well plate before 24 hours. After 24 hours, in order to sensitize IgE, a dilution obtained by serially diluting 10 nM IgE in blocker IgE from the starting concentration of 20 nM in 400 µl of DMEM containing no fetal bovine serum, penicillin and streptomycin was added thereto, followed by culturing for 3 hours. The cells were washed twice with 500 ul of Siraganian buffer and were allowed to stand in 160 µl Siraganian buffer in a 5% CO₂, 37°C incubator for 10 minutes. 140 nM of recombinant biotinylated Protein L (Thermo Fisher Scientific; Cat# 29997) and 70 nM of streptavidin-fluorescein (Vector Laboratories; Cat# SA-5001) were mixed with Siraganian buffer, 20 µl of the resulting mixture was added to each well and then the result was allowed to stand for 20 minutes. 0.1% Triton X-100 used as a control group was incubated for 1 hour in a 5% CO₂, 37°C incubator. After incubation in a 5% CO₂, 37°C incubator for 20 minutes, 50 µl of the result was added to each well of a 96-well polystyrene plate, and 50 µl of 1 mM p-NAG (p-nitrophenyl N-acetyl-beta-D-glucosamine in 0.1 M citrate buffer, pH 4.5) was added thereto. After treatment at 37°C for 1 hour, 200 µl of stop solution (0.1 M Na₂CO₃/NaHCO₃, pH 10.0) was added to each well and absorbance at 405 nm was measured using a microplate reader (Molecular Devices Inc.).

### Example 3: Confirmation of IgCw-γεκ purification yield

The yields of immunoglobulin molecules produced by transient transfection of HEK293F cells with KV10-IgCw-γεκ, KV12-6C407 IgE, KV10-IgCw-εκ, and plasmids, and suspension culture were compared (Table 1). The predicted structure of the expressed proteins is shown in FIG. 1. 7 days after transfection, all proteins were purified using Kappa XP-Agarose which binds to the human kappa constant region. IgCw-εκ including only the epsilon heavy-chain and the kappa light-chain constant region did not form a disulfide bond between the heavy-chain and the light-chain constant regions, and thus only the kappa constant region could be purified (FIG. 2). On the other hand, the antibody fragment IgCw-γεκ which includes the constant regions of the heavy- and light-chains in which the gamma constant region (Cγ1) is fused with the epsilon constant region (Cε₂₋₄) has a disulfide bond normally formed between the constant regions and has a similar expression level to control 6C407 IgE (FIG. 2), an average yield of 17 mg/L, which is identical to 6C407 IgE and has an average yield of ~27 mg/L when purified on KappaXP-agarose (Table 1). This indicates that the IgCw-γεκ protein is structurally more stable and has a higher purification yield than the IgCw-εκ protein.

**[Table 1]**

| Antibody (fragment) protein yield obtained from HEK293F cell culture | | |
|---|---|---|
| Antibody protein | Affinity chromatography | Average yield (mg/L) |
| 6C407 IgE | CaptureSelect^{™} KappaXP-agarose | 17 |
| IgCw-εκ | CaptureSelect^{™} KappaXP-agarose | 1 |
| IgCw-γεκ | CaptureSelect^{™} KappaXP-agarose | 17 |
| | CaptureSelect^{™} IgG-C_{H}1-agarose | 27 |

The purification yield of the IgCw-γεκ protein is similar to or higher than that of IgE depending on the purification method.

### Example 4: Confirmation of expression of human Fcε receptor in RBL-2H3 cells

Flow cytometry was performed to determine whether or not hFcεR is expressed in RBL-2H3-hFcεRIα cells expressing the human Fcε receptor.

The anti-Fcε antibody did not bind to wild-type RBL-2H3 cells, but bound to RBL-2H3-hFcεRIα cells expressing the Fcε receptor (FIG. 3). This means that the human Fcε receptor is not expressed in wild-type RBL-2H3 cells and exists only in RBL-2H3-hFcεRIα cells expressing the human Fcε receptor.

### Example 5: Confirmation of binding of IgCw-γεκ to Fcε receptor and background signal

Flow cytometry was performed to determine whether or not IgCw-γεκ binds to Fcε receptors of cells expressing human Fcε receptors on the surface thereof.

IgCw-γεκ binds to RBL-2H3-hFcεRIα cells formed to express hFcεRIα like full-size IgE, but does not bind to RBL-2H3 cells lacking any hFcεRIα (FIG. 3). This means that IgCw-γεκ can recognize Fcε receptors like full-size IgE.

### Example 6: Use of IgCw-γεκ as reference for degranulation experiments using IgE

Whether or not IgCw-γεκ could be used instead of full-size IgE in an experiment using IgE, such as a degranulation experiment, was determined by a beta-hexosaminidase secretion experiment.

Experiments were performed with polyclonal IgE and 6C407 IgE as well as 6C407 IgG and PBS as controls. Secretion of beta-hexosaminidase from RBL-2H3-hFcεRIα cells was not observed in negative controls 6C407 IgG and PBS, and secretion of beta-hexosaminidase was observed only in IgE-treated samples (FIG. 4). IgCw-γεκ exhibited secretion percentages similar to or higher than full-size IgE, suggesting that the function of IgCw-γεκ was similar to full-size IgE. This means that IgCw-γεκ can be used as a reference molecule in degranulation experiments instead of human IgE.

### Example 7: Use of IgCw-γεκ as blocker of Fcε receptors

Whether or not IgCw-γεκ could be used as a blocker for the Fcε receptor was determined.

IgCw-γεκ cannot be bind to protein L because it does not have a variable region. Therefore, activation signals of RBL-2H3-hFcεRIα cells can be observed only when full-size IgE binds to Fcε. When RBL-2H3-hFcεRIα cells were treated with a mixture of 6C407 IgE and IgCw-γεκ (Fig. 5B), as well as when RBL-2H3-hFcεRIα cells were pre-treated with 6C407 IgE and then treated with IgCw-γεκ (Fig. 5C), as the IgCw-γεκ concentration increased, the signal for 6C407 IgE decreased (FIG. 5). This means that IgCw-γεκ interfered with the binding of full-size IgE to the Fcε receptor, indicating that IgCw-γεκ could be used as an Fcε receptor blocker.

### Example 8: Use of IgCw-γεκ as reference for IgG concentration quantification

Whether or not IgCw-γεκ could be used as a substitute for full-size IgE which is a reference used to determine IgG concentration was determined.

Two logarithmic standard curves were created by enzyme-linked immunosorbent assay (ELISA) using known concentrations of human polyclonal IgE (FIG. 6A) and IgCw-γεκ (FIG. 6B), respectively. The same experiment was performed using two samples of monoclonal IgE of unknown concentration (6C407 and 3D8). The IgE concentration was determined by interpolation in each curve (Table 2) and the linear interpolation formula used herein (https://formulas.tutorvista.com/math/interpolation-formula.html) was x = x₁ + (x₂ - x₁) × (y - y₁) / (y₂ - y₁).

**[Table 2]**

| IgE concentration measured using two standard curves | | | | |
|---|---|---|---|---|
| Sample of unknown concentration | Absorbance at 405 nm | Concentration measured by standard curve using each reference protein (ng/ml) | | |
| | | Human IgE | IgCw-γεκ | |
| | | | Raw data | Corrected data (x 1.462)* |
| 6C407 IgE | 0.466 | 70.42 | 51.55 | 75.366 |
| 3D8 IgE | 0.189 | 25.01 | 16.92 | 24.737 |

| | | | | |
|---|---|---|---|---|
| *IgE concentration determined by IgCw-γεκ standard curve was multiplied by the ratio of molecular weight (molecular weight ratio of IgE (~190kDa): IgCw-γεκ(~130 kDa)=1.462:1) for correction. | | | | |

The normalized concentrations of 6C407 IgE and 3D8 IgE were 75.366 ng/ml and 24.737 ng/ml, respectively, which are very similar to the concentrations (6C407 IgE: 70.42 ng/ml, 3D8 IgE: 25.01 ng/ml) determined by a standard curve using human polyclonal antibodies. This indicates that IgCw-γεκ can be used as a reference molecule to determine human IgE concentrations.

### [Industrial applicability]

The antibody fragment according to the present invention can be widely used as a substitute for IgE reference antibodies used in various immunological studies. The antibody fragment can be used both as a reference molecule to measure the concentration of IgE antibody in a sample and an Fc epsilon receptor (FcεRI) blocker to suppress type 1 allergic reactions.

In addition, the antibody fragment according to the present invention can be used as a blocker to suppress an autoimmune reaction associated with an autoantibody of the IgE isotype.

In addition, the antibody fragment according to the present invention does not contain a variable region and thus completely avoids the non-specific cross-reaction, that is, the off-target effect due to reaction with other antigens, which is a disadvantage of conventional full-length isotype control antibodies, thus being more suitably used in quantitative and qualitative experiments of IgE, and used as an isotype control antibody to evaluate the activity of experimental/therapeutic antibodies.

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

### [Sequence Listing Free Text]

An electronic file is attached.

## Claims

1. An antibody fragment comprising:
a heavy-chain constant region fragment of an IgG antibody and a heavy-chain constant region fragment of an IgE antibody; and
a light-chain constant region fragment linked to the heavy-chain constant region fragment.

2. The antibody fragment according to claim 1, wherein the antibody fragment does not comprise variable domains.

3. The antibody fragment according to claim 1, wherein the heavy-chain constant region fragment of the IgG antibody comprises C_{γ1}.

4. The antibody fragment according to claim 1, wherein the heavy-chain constant region fragment of the IgE antibody comprises at least one selected from the group consisting of Cε2, Cε3, and Cε4.

5. The antibody fragment according to claim 1, wherein the heavy-chain constant region fragment of the IgG antibody is linked to the heavy-chain constant region fragment of the IgE antibody by a hinge.

6. The antibody fragment according to claim 1, wherein the IgG antibody heavy-chain constant region fragment is linked to the IgE antibody heavy-chain constant region fragment from an N-terminus to a C-terminus in a form of Cγ1-Cε2-Cε3-Cε4.

7. The antibody fragment according to claim 1, wherein the light-chain constant region comprises Cκ or CX.

8. The antibody fragment according to claim 1, wherein the light-chain constant region fragment linked to the heavy-chain constant region fragment is bonded thereto by a disulfide bond or via a peptide linker.

9. A nucleic acid encoding the antibody fragment according to any one of claims 1 to 8.

10. A kit comprising the antibody fragment according to any one of claims 1 to 8.

11. A composition for evaluating antibody efficacy comprising the antibody fragment according to any one of claims 1 to 8.

12. A composition for measuring an antibody concentration comprising the antibody fragment according to any one of claims 1 to 8.

13. A composition for suppressing allergic reactions comprising the antibody fragment according to any one of claims 1 to 8.

14. A composition for inhibiting an IgE-mediated-autoimmune reaction comprising the antibody fragment according to any one of claims 1 to 8.
